# EUROPEAN PATENT APPLICATION

(11) **EP 4 736 939 A2**
(43) Date of publication of application: **06.05.2026**
(21) Application number: 25213496.0
(22) Date of filing: 04.11.2025
(51) Int. Cl.: A61N 5/06, A61C 19/06

(54) **ORAL CARE DEVICE**

(30) Priority: 04.11.2024 CN 202422679925 U
(71) Applicant: Shenzhen Oren Medical Technology Co., Ltd., Shenzhen, Guangdong 518103 (CN)
(72) Inventor: OU, Baihui, Shenzhen, 518103 (CN); OU, Bailing, Shenzhen, 518103 (CN); YU, Zhang, Shenzhen, 518103 (CN); DIJKSTRA, Alain, 1186 GK Amstelveen (NL)
(74) Representative: Valet Patent Services Limited

(57) **Abstract**

Embodiments of the present invention provide a multifunctional oral care device comprising a lip care assembly and a tooth care assembly that can be detachably connected or used independently. The assemblies include light therapy components configured to emit specific wavelengths for lip and oral treatment. The device further includes an orientation detection module for mode switching, dual or synchronized control units with detachable power supplies, and optional intermediate layers for irradiating inner lip surfaces. A nasal care phototherapy device can also be connected through an auxiliary interface for combined facial or oral therapy.

## Description

### TECHNICAL FIELD

The present invention relates to beauty and personal care devices, and more particularly to an oral care or mouth beauty device configured for cosmetic or therapeutic treatment of lips, teeth, and surrounding oral tissues.

### BACKGROUND ART

Lip beauty devices are commonly used in the field of cosmetic and oral care technology to enhance the appearance and health of the lips. Such devices generally employ light-based or phototherapy techniques, wherein light of specific wavelengths is directed toward the lips to stimulate collagen production, improve blood circulation, and promote skin rejuvenation. As a result, regular use of these devices can lead to firmer, smoother, and more vibrant lips.

In conventional designs, a lip beautification device typically requires a bite or occlusal structure positioned inside the mouth, which the user grips with the teeth to hold the device in place during use. This structure provides mechanical support and maintains the proper position of the lip treatment portion relative to the lips and mouth. However, existing devices suffer from several limitations that reduce user comfort, treatment stability, and functional reliability.

Most known lip beautifiers and occlusal assemblies employ magnetically coupled or electrode-based connection mechanisms between the lip treatment portion and the occlusal (tooth-supporting) portion. These magnetic or electrode connections are often insufficiently stable, particularly during extended use or when the user moves the jaw or lips. The coupling may easily become loose or detached, causing the device to fall from the user's mouth or leading to interruption of the electrical circuit that powers the light sources. This instability not only disrupts the phototherapy process but also degrades the overall user experience.

Moreover, conventional devices are typically designed as single-function systems, operating either as lip treatment devices or as tooth whitening or oral therapy devices. This limits their usability and fails to provide comprehensive treatment across the lip and oral regions. Users seeking simultaneous treatment of lips, teeth, or inner oral tissues are therefore required to employ multiple, separate devices, resulting in inconvenience and inefficiency.

Furthermore, existing devices generally lack intelligent control coordination between multiple treatment assemblies. For example, when lip and tooth treatment units are combined, there is often no effective mechanism for synchronized operation, independent mode control, or automated switching between treatment states based on the user's position or orientation of the device. Such limitations restrict the adaptability and precision of light-based treatments within the oral cavity.

Accordingly, there remains a need for an improved oral beauty device that provides a stable and reliable connection between lip and tooth treatment assemblies, ensures uninterrupted electrical communication during use, and enables both independent and coordinated operation of multiple phototherapy circuits. It is also desirable for such a device to support modular expansion, allowing detachable integration with additional therapy assemblies, such as nasal treatment modules, and to incorporate intelligent detection or control functions for optimizing treatment performance and user comfort.

### OBJECTS OF THE INVENTION

Some of the objects of the invention are as follows:
An object of the present invention is to provide an oral care or mouth beauty device capable of performing cosmetic and therapeutic phototherapy on the lips, teeth, and adjacent oral tissues in a coordinated and efficient manner.

Another object of the invention is to provide an oral care device having a lip care assembly and a tooth care assembly that can be mechanically and electrically coupled or detached, thereby allowing both assemblies to operate either independently or in a synchronized phototherapy mode when connected.

A further object of the invention is to provide a stable and reliable detachable coupling structure between the lip care assembly and the tooth care assembly, ensuring a firm mechanical connection and uninterrupted electrical communication during use, while maintaining user comfort and ease of attachment or removal.

Yet another object of the invention is to provide an oral care device incorporating independent control units or controllers for the lip care and tooth care assemblies, enabling autonomous operation of each module when used separately, and master-slave coordinated control when the assemblies are coupled together.

An additional object of the invention is to provide a modular oral care system that can be expanded to include additional therapeutic assemblies, such as a nasal therapy module, thereby allowing comprehensive beauty and wellness treatment for multiple facial regions using a single integrated system.

Another object of the invention is to provide an oral care device having an intermediate light-emitting layer positioned between the lip and tooth assemblies, enabling simultaneous irradiation of the inner and outer lip surfaces for improved cosmetic and therapeutic effects.

A further object of the invention is to provide a device incorporating a load or orientation detection module capable of sensing the relative position of the tooth care assembly and automatically selecting or adjusting the operational mode of one or more phototherapy circuits based on the detected orientation.

Yet another object of the invention is to provide a phototherapy-based oral beauty device that enhances user safety, comfort, and treatment efficiency, while reducing the likelihood of electrical disconnection, slippage, or misalignment during operation.

Yet another object of the present invention is to provide an intelligent, user-friendly, and multifunctional oral care device that combines the benefits of light-based lip care, tooth whitening, and oral therapy within a compact, detachable, and aesthetically designed form factor.

### SUMMARY OF THE INVENTION

According to a first aspect of the present invention, an oral care device is provided. The oral care device comprising: a lip care assembly including a first phototherapy circuit configured to emit light for cosmetic or therapeutic treatment of lips, and a first electrical contact; a tooth care assembly including a second phototherapy circuit configured to emit light for cosmetic or therapeutic treatment of teeth, and a second electrical contact; at least one control unit configured to control operation of the oral care device; and a detachable coupling structure configured to mechanically and electrically connect the lip care assembly and the tooth care assembly; wherein electrical communication between the first electrical contact and the second electrical contact enables coordinated or independent operation of the first phototherapy circuit and the second phototherapy circuit.

In one embodiment of the invention, the at least one control unit comprises: a first controller and a first power supply associated with the lip care assembly; and a second controller and a second power supply associated with the tooth care assembly.

In one embodiment of the invention, the lip care assembly is configured to operate independently under control of the first controller and the first power supply when uncoupled from the tooth care assembly; the tooth care assembly is configured to operate independently under control of the second controller and the second power supply when uncoupled from the lip care assembly; and when the lip care assembly and the tooth care assembly are mechanically and electrically coupled, the first controller and the second controller are configured to operate in a coordinated phototherapy mode under synchronized control.

In one embodiment of the invention, when the tooth care assembly is coupled to the lip care assembly, the first controller automatically operates in a master-control mode to coordinate and control the operation of the second controller and the second phototherapy circuit.

In one embodiment of the invention, the oral care device includes a single control unit that is detachably attached to, or disposed within, the housing of the lip care assembly, and is configured to operate both the lip care assembly and the tooth care assembly.

In one embodiment of the invention, the oral care device further comprising: a nasal therapy assembly configured to be detachably connected to the at least one of the lip care assembly or the tooth care assembly for cosmetic or therapeutic treatment of nasal tissue.

In one embodiment of the invention, at least one of the control units is disposed within the lip care assembly or is detachably attachable to the lip care assembly.

In one embodiment of the invention, the detachable coupling structure comprises: a first support portion extending from the lip care assembly; and a second support portion extending from the tooth care assembly and detachably connectable with the first support portion to mechanically couple the lip care assembly and the tooth care assembly; the first electrical contact disposed on the lip care assembly and electrically connected to the first phototherapy circuit, and a second electrical contact disposed on the tooth care assembly and electrically connected to the second phototherapy circuit; wherein, when the first support portion and the second support portion are connected, the first electrical contact and the second electrical contact engage to establish electrical communication between the lip care assembly and the tooth care assembly for synchronized or shared-power operation.

In one embodiment of the invention, the first support portion comprises a support column, and the second support portion comprises a support groove configured to receive the support column to enable insertion and mechanical coupling between the lip care assembly and the tooth care assembly. When engaged, these portions establish both mechanical coupling and electrical communication. At least one of the support portions or electrical contacts includes magnetic coupling elements to ensure proper alignment and secure electrical connection between the assemblies.

In one embodiment of the invention, the at least one of the first support portion and the second support portion, or the first electrical contact and the second electrical contact, includes a magnetic coupling structure configured to provide mechanical alignment and electrical connection between the lip care assembly and the tooth care assembly.

In one embodiment of the invention, the tooth care assembly comprises a second mount including an occlusal sleeve and a second bracket disposed within the occlusal sleeve; and the second bracket comprising a first tooth support and a second tooth support, and the second phototherapy circuit is supported by the second bracket.

In one embodiment of the invention, the tooth care assembly includes an occlusal sleeve and a second bracket having a first and second tooth support. The second phototherapy circuit disposed between the occlusal sleeve and the tooth support to emit light toward both inner-lip tissue and the teeth. The coupling interface and electrodes positioned at an exposed end of the bracket adjacent the lip care assembly for efficient connectivity.

In one embodiment of the invention, an end of the second bracket is exposed from the occlusal sleeve adjacent the lip care assembly, and the second support portion and the second electrical contacts are positioned at the exposed end of the second bracket.

According to a second aspect of the present invention, an oral care device is provided. The oral care device comprising: a lip care assembly including a first light-emitting layer configured to emit light within a first wavelength range suitable for enhancing lip appearance; a tooth care assembly including a second light-emitting layer configured to emit light within a second wavelength range suitable for treating teeth or oral tissue; and an intermediate layer disposed between the lip care assembly and the tooth care assembly, the intermediate layer being positioned to contact or face an inner surface of lips and comprising a third light source configured to emit light having a wavelength effective for treating inner-lip tissue or sores; wherein, during use, lips are positioned between the first light-emitting layer and the intermediate layer, enabling simultaneous irradiation of inner and outer lip surfaces.

In one embodiment of the invention, the intermediate layer is disposed on the backside of the tooth care assembly, the backside being oriented toward an inner surface of the lips when the tooth care assembly is positioned within a mouth.

In certain embodiments of the invention, the intermediate layer positioned between the lip care assembly and the tooth care assembly. This layer comprises a third light source configured to emit light for treating inner-lip tissue or sores, allowing simultaneous irradiation of both the inner and outer lip surfaces.

According to a third aspect of the present invention, an oral care device is provided. The oral care device comprising: a lip care assembly including a first electrical contact and a first phototherapy circuit; a tooth care assembly including a second electrical contact and a second phototherapy circuit; and a load detection module electrically connected to at least one of the phototherapy circuits, the load detection module being configured to detect a positional state of the tooth care assembly relative to the lip care assembly based on electrical loading between corresponding electrical contacts, and to selectively operate one or more of the phototherapy circuits according to the detected positional state.

In one embodiment of the invention, the load detection module is configured to activate the first phototherapy circuit when the tooth care assembly is in a first orientation and to activate the second phototherapy circuit when the tooth care assembly is in a second orientation.

In one embodiment of the invention, the load detection module is configured to detect an orientation of the tooth care assembly and to control an operational mode of the first phototherapy circuit and the second phototherapy circuit based on a detected positional state of the tooth care assembly.

In the context of the specification, when an element is referred to as being "fixed to" or "disposed to" another element, it may either be directly on another element or indirectly on that other element. When a component is said to be "connected" or "connected to" another component, it may be directly connected to another component or indirectly connected to other components on the piece.

In the context of the specification, the terms "first", "second," and "third" are only used for descriptive purposes and do not imply the relative importance or implicitly indicate the quantity of technical features indicated.

In the context of the specification, the term "plurality" means two or more than two, unless otherwise indicated.

In the context of the specification, the term "several" means more than one, unless otherwise specified.

In the context of the specification, the term "phototherapy element/component/ light panel" encompasses any light-emitting device capable of emitting light of therapeutic wavelength(s), including but not limited to light-emitting diodes (LEDs), organic LEDs (OLEDs), laser diodes, or equivalent optical sources. The light may include ultraviolet, visible, near-infrared, or far-infrared spectra.

In the context of the specification, the term "control unit" refers to any input or output mechanism enabling a user to operate the device. The control unit may include physical buttons, capacitive touch sensors, sliders, switches, or graphical displays, and may further include wireless control via a mobile application.

In the context of the specification, the term "circuit board" encompasses any printed circuit board (PCB), flexible circuit, or equivalent substrate that supports and electrically connects components of the device, including power supplies, control chips, drivers, or stimulation elements.

In the context of the specification, the term "user" or "subject" is intended to broadly cover humans, animals, or other recipients of the treatment, unless otherwise specifically limited.

In the context of the specification, the term "LED module" refers to one or more light-emitting diode (LED) elements that are electrically connected and configured to emit light of specific wavelengths suitable for therapeutic purposes. The LED module may include drive circuitry, heat dissipation structures, and optical elements such as lenses or diffusers to control light distribution.

In the context of the specification, the term "light source" or "phototherapy source" etc. refers to a source emitting coherent laser light, or light-emitting diodes ("LEDs"). The term "light therapy" refers to light generated from any of the sources, such as lasers, LED sources, or Super luminous diodes ("SLD").

In the context of the specification, "Light Emitting Diodes (LEDs)" refer to semiconductor diodes capable of emitting electromagnetic radiation when supplied with an electric current. The LEDs are characterized by superior power efficiencies, smaller sizes, rapid switching speeds, physical robustness, and longer lifespans compared to incandescent or fluorescent lamps. The one or more LEDs may include through-hole type LEDs (generally emitting electromagnetic radiation in red, green, yellow, blue, and white colors), Surface Mount Technology (SMT) LEDs, Bi-color LEDs, Pulse Width Modulated RGB (Red-Green-Blue) LEDs, and high-power LEDs, among others.

Materials used in one or more LEDs may vary from one embodiment to another, depending upon the frequency of radiation required. Different frequencies can be obtained from LEDs made from pure or doped semiconductor materials. Commonly used semiconductor materials include nitrides of Silicon, Gallium, Aluminum, Boron, Zinc Selenide, etc., in pure form or doped with elements such as Aluminum and Indium. For example, red and amber colors are produced from Aluminum Indium Gallium Phosphide (AlGaInP) based compositions, while blue, green, and cyan use Indium Gallium Nitride based compositions. White light may be produced by mixing red, green, and blue lights in equal proportions, while varying proportions may be used to generate a wider color gamut. White and other colored lightings may also be produced using phosphor coatings such as Yttrium Aluminum Garnet (YAG) in combination with a blue LED to generate white light, and Magnesium-doped potassium fluorosilicate in combination with a blue LED to generate red light.

In addition to conventional mineral-based LEDs, one or more LEDs may also be provided on an Organic LED (OLED) based flexible panel or an inorganic LED-based flexible panel. Such OLED panels may be generated by depositing organic semiconducting materials over Thin Film Transistor (TFT) based substrates. Further, a discussion on the generation of OLED panels can be found in Bardsley, J. N (2004), "International OLED Technology Roadmap", IEEE Journal of Selected Topics in Quantum Electronics, Vol. 10, No. 1, that is included herein in its entirety, by reference. An exemplary description of flexible inorganic light-emitting diode strips can be found in granted U.S. Pat. No. 7,476,557 B2, titled "Roll-to-roll fabricated light sheet and encapsulated semiconductor circuit devices", which is included herein in its entirety by reference.

In the context of this specification, terms like "light", "radiation", "irradiation", "emission" and "illumination", etc. refer to electromagnetic radiation in frequency ranges varying from the Ultraviolet (UV) frequencies to Infrared (IR) frequencies and wavelengths, wherein the range is inclusive of visible light, UV and IR frequencies and wavelengths. It is to be noted here that UV radiation can be categorized in several ways depending on respective wavelength ranges, all of which are envisaged to be under the scope of this invention. For example, UV radiation can be categorized as Hydrogen Lyman-α (122-121 nm), Far UV (200-122 nm), Middle UV (300-200 nm), and Near UV (400-300 nm). The UV radiation may also be categorized as UVA (400-315 nm), UVB (315-280 nm), and UVC (280-100 nm). Similarly, IR radiation may also be categorized into several categories according to respective wavelength ranges, which are again envisaged to be within the scope of this invention. A commonly used subdivision scheme for IR radiation includes Near IR (0.75-1.4 µm), Short-Wavelength IR (1.4-3 µm), Mid-Wavelength IR (3-8 µm), Long-Wavelength IR (8-15 µm), and Far IR (15-1000 µm).

Unless otherwise stated, the term "light" as used in this specification encompasses electromagnetic radiation in the visible (380-780 nm) and infrared (780 nm-1000 nm) ranges, particularly red light (620-750 nm) and near-infrared (750-1400 nm) wavelengths commonly used in photobiomodulation therapy. Particular wavelengths which may be selected as the dominant emissive wavelength may include the follow, without any preference to be indicated by order: 400 nm, 405 nm, 420 nm, 430 nm, 450 nm, 465 nm, 515 nm, 530 nm, 532 nm, 590 nm, 630 nm, 633 nm, 640 nm, 650 nm, 655 nm, 660 nm, 670 nm, 680 nm, 780 nm, 785 nm, 810 nm, 830 nm, 840 nm, 850 nm, 860 nm, 870 nm, 904 nm, 915 nm, 980 nm, 1015 nm, 1060 nm, 1065 nm, 1070 nm, 1200, and 1400 nm. As used herein, the term "light therapy" refers to the use of one or more light sources of any type that emit light with a wavelength between about 400 and 1400 nm. The device may also emit blue or ultraviolet light for surface-level treatments such as acne reduction or microbial control.

The red light (approximately 630-660 nm) penetrates deeply into the scalp to stimulate blood circulation and enhance hair follicle activity, thus promoting hair growth and repair. Blue light (around 415-470 nm) exhibits antibacterial properties and is effective in treating scalp acne and reducing inflammation. Green light (approximately 520-540 nm) can help reduce pigmentation and soothe sensitive or irritated scalp tissue. Yellow light (around 580-600 nm) improves oxygen exchange in the cells and aids in detoxifying the scalp, while near-infrared light (800-850 nm) reaches deeper layers to accelerate healing and reduce pain.

### BRIEF DESCRIPTION OF THE ACCOMPANYING DRAWINGS

The accompanying drawings illustrate the best mode for carrying out the invention as presently contemplated and set forth hereinafter. The present invention may be more clearly understood from a consideration of the following detailed description of the preferred embodiments taken in conjunction with the accompanying drawings, wherein like reference letters and numerals indicate the corresponding parts in various figures in the accompanying drawings, and in which:
**FIG. 1** is a schematic structural diagram of an oral care device comprising a lip care assembly and a tooth care assembly, in accordance with an embodiment of the present invention.
**FIG. 2** shows a sectional view taken along line A-A of **FIG. 1****,** in accordance with an embodiment of the present invention.
**FIG. 3** shows an exploded view illustrating the structure of the lip care assembly, in accordance with an embodiment of the present invention.
**FIG. 4** is a schematic structural diagram illustrating the configuration of the tooth care assembly, in accordance with an embodiment of the present invention.
**FIG. 5** shows an exploded view of the tooth care assembly, in accordance with an embodiment of the present invention.
**FIG. 6** shows an enlarged view of portion B shown in **FIG. 2****,** in accordance with an embodiment of the present invention.
**FIG. 7** is an exploded view illustrating the connection relationship between the lip care assembly, the tooth care assembly, and a mouthpiece, in accordance with an embodiment of the present invention.
**FIG. 8** is a schematic structural diagram illustrating the configuration of the mouthpiece in an exemplary embodiment of the present invention.
**FIG. 9** is a schematic view illustrating an example embodiment of the present invention in which the lip care assembly is connected to the mouthpiece, in an exemplary embodiment of the present invention.
**FIG. 10** is an exploded view illustrating the connection between the tooth care assembly and a charging assembly, in accordance with an embodiment of the present invention.
**FIG. 11** is an enlarged view of portion C shown in **FIG. 10****,** in accordance with an embodiment of the present invention.
**FIG. 12A** illustrates a schematic view of a lip beautification assembly including four electrodes configured for connection with corresponding terminals of a tooth care assembly to enable mode selection and load/orientation detection.
**FIG. 12B** illustrates a circuit diagram of a load and orientation detection module, showing connection terminals and light-emitting diodes (LED1, LED2) for selectively activating different operating modes of the tooth care assembly.
**FIG. 13** illustrates an exploded perspective view of a tooth care assembly showing an intermediate layer configured to irradiate the inner side of the lip.
**FIG. 14** illustrates the oral care device having an intermediate phototherapy layer 236 to irradiate inner surface of lips.
**FIG. 15A** illustrates a perspective view showing a first control unit including a controller and a power supply detachably connected to the tooth care assembly.
**FIG. 15B** illustrates a perspective view showing a second control unit detachably connected to the lip care assembly, wherein the control units may operate in a synchronized or master-slave configuration, or may be implemented as a single integrated control unit.
**FIG. 16** illustrates a nasal care phototherapy device connected to the control unit through an auxiliary connector cable, enabling modular operation and synchronized phototherapy with other treatment assemblies such as lip or tooth care modules.

### DETAILED DESCRIPTION

Embodiments of the present invention disclosure will be described more fully hereinafter with reference to the accompanying drawings in which like numerals represent like elements throughout the figures, and in which example embodiments are shown.

The detailed description and the accompanying drawings illustrate the specific exemplary embodiments by which the disclosure may be practiced. These embodiments are described in detail to enable those skilled in the art to practice the invention illustrated in the disclosure. It is to be understood that other embodiments may be utilized, and other changes may be made, without departing from the spirit or scope of the present disclosure. The following detailed description is therefore not to be taken in a limiting sense, and the scope of the present invention disclosure is defined by the appended claims. Embodiments of the claims may, however, be embodied in many different forms and should not be construed as limited to the embodiments set forth herein.

The terms "a" and "an" herein do not denote a limitation of quantity, but rather denote the presence of at least one of the referenced items. The terms "having", "comprising", "including", and variations thereof signify the presence of a component.

Embodiments of the present invention disclose an oral care device designed for both cosmetic and therapeutic treatment of the lips and oral cavity. The device generally comprises a lip care assembly and a tooth care assembly that can be detachably connected to each other. The lip care assembly is configured to emit light for the enhancement of lip appearance and condition, while the tooth care assembly emits light for dental whitening, cleaning, or oral tissue treatment. The two assemblies are interconnected through a coupling structure that provides both mechanical connection and electrical communication, thereby allowing coordinated or independent operation depending on user preference.

In an embodiment, each assembly includes its own controller and power supply for standalone use, while a master-control mode allows synchronized operation when coupled.

In one embodiment, the coupling structure includes a first support portion extending from the lip care assembly and a second support portion extending from the tooth care assembly. The first support portion is configured to take the form of a support column or projection, while the second support portion defines a complementary groove or recess for receiving the projection. This configuration provides a stable and firm mechanical engagement between the assemblies. The electrical communication between the assemblies is achieved by corresponding electrical contacts positioned at the connection interface. When the two assemblies are coupled, the electrical contacts align and engage, thereby forming a conductive path for the transfer of power and control signals. In certain configurations, the electrical contacts or support portions are magnetically assisted to facilitate automatic alignment and prevent accidental disconnection during use. Such magnetic coupling enhances both mechanical stability and circuit continuity.

The lip care assembly comprises a housing that accommodates a phototherapy circuit, a control circuit, and optionally a power source. The phototherapy circuit includes a plurality of light-emitting diodes or other light sources configured to emit light in wavelength ranges effective for stimulating collagen production, enhancing blood circulation, and improving the texture and color of the lips. The control circuit regulates the operation of the light sources, including their emission intensity, timing, and mode of operation. The control circuit is configured to operate autonomously when the lip care assembly is used independently or operate in synchronization with the control circuit of the tooth care assembly when the two assemblies are connected.

The tooth care assembly comprises an occlusal portion adapted to be comfortably placed between the upper and lower teeth of a user. The occlusal portion includes one or more brackets or supports configured to hold a phototherapy circuit for dental treatment. The phototherapy circuit of the tooth care assembly includes light sources that emit blue or voilet light suitable for tooth whitening or therapeutic effects on oral tissue. The light sources are generally positioned such that emitted light reaches both the teeth and the inner surface of the lips, allowing simultaneous treatment of multiple oral regions. The exposed end of the tooth care assembly extends outward to form part of the coupling interface for attachment to the lip care assembly.

In another embodiment, an intermediate light-emitting layer is provided between the lip care assembly and the tooth care assembly. The intermediate layer includes an additional phototherapy circuit configured to emit light toward the inner surface of the lips. When the device is in use, the outer surface of the lips is irradiated by the light emitted from the lip care assembly, while the inner surface of the lips receives light from the intermediate layer. This dual-surface illumination enables uniform treatment across the entire lip tissue and enhances cosmetic effectiveness. The intermediate layer is either formed integrally with the tooth care assembly or configured as a detachable module.

In alternate embodiments, the intermediate layer positioned between the lip and tooth assemblies includes a third light source configured to irradiate inner-lip tissue, enabling simultaneous treatment of inner and outer lip surfaces.

In one embodiment of the present invention, the first phototherapy circuit emits red and infrared light for lip treatment, while the second phototherapy circuit emits blue and violet light for tooth treatment.

In an embodiment of the present invention, the first phototherapy circuit and/or the second phototherapy circuit includes a micro-current element, an electrotherapy element, a heating element, a cooling element, or a vibration element, either present individually or in combination.

The oral care device further includes an optional nasal therapy module configured for attachment to either the lip care assembly or the tooth care assembly. The nasal module includes one or more nasal light emitters designed to deliver light therapy to the nasal region to promote skin rejuvenation or relieve inflammation. This modular arrangement allows the device to be extended for multiple treatment purposes, forming a multi-functional beauty and wellness system.

In certain embodiments, the oral care device incorporates at least one control unit that manages the operation of both the lip and tooth assemblies. Each assembly includes its own controller and power source, allowing independent operation when used separately. When connected, one controller acts as a master controller to coordinate the operation of both assemblies. Synchronization between controllers ensures balanced light output, proper wavelength combination, and consistent treatment cycles across the connected modules.

The oral care device further comprises a load detection or orientation sensing module configured to detect the position or engagement state between the lip and tooth assemblies. The detection module determines the operational state of the device based on electrical load variations or mechanical contact conditions. Depending on the detected configuration, the device automatically activates the lip care mode, tooth care mode, or combined dual-mode operation. This intelligent control mechanism simplifies operation, enhances safety, and ensures that light emission occurs only under proper engagement conditions.

The device also includes a charging assembly configured to provide electrical energy to the controllers and power circuits. The charging assembly connects directly to the tooth care assembly through an electrical interface or is designed for wireless charging. In some examples, the charging assembly also serves as a docking station that facilitates sterilization and safe storage of the device when not in use.

Through the above configurations, the oral care device achieves improved functional integration between lip care and oral treatment modules. The detachable connection ensures stable mechanical engagement and reliable electrical communication, preventing accidental detachment or power interruption during operation. The independent or synchronized control of multiple light sources allows flexible use according to treatment needs. The provision of an intermediate light-emitting layer enables dual-surface irradiation for enhanced cosmetic results, while the modular expansion design allows the addition of further therapeutic components such as nasal treatment modules. The inclusion of load detection and intelligent control provides safety and ease of use, while the integrated charging structure offers convenience and portability. Overall, the present invention provides a comprehensive and user-friendly oral care system capable of performing multiple beauty and therapeutic functions with high stability, comfort, and efficiency.

The oral care device disclosed in the present invention provides significant improvements over conventional lip beautifiers and oral treatment devices. Through the coordinated integration of a lip care assembly and a tooth care assembly, the device enables comprehensive phototherapy of both the lips and the oral cavity within a single, modular structure. The detachable coupling structure ensures reliable mechanical stability and uninterrupted electrical communication, effectively overcoming the problem of loose or unstable magnetic connections found in conventional devices. This design prevents accidental disconnection and ensures continuous power supply and signal transmission during use.

By enabling independent or synchronized operation of the lip and tooth assemblies, the invention allows users to selectively perform lip treatment, tooth treatment, or combined therapy according to personal needs. The inclusion of separate or coordinated controllers provides intelligent power management and flexible operation modes, while the master-slave control architecture ensures synchronized light emission and uniform phototherapeutic effects when the assemblies are interconnected.

The configuration of an intermediate light-emitting layer positioned between the lip and tooth assemblies allows simultaneous treatment of the inner and outer surfaces of the lips. This dual-sided irradiation enhances the therapeutic and cosmetic effect, improving collagen stimulation, lip firmness, and overall appearance. Furthermore, the modular design permits integration of additional functional units, such as a nasal therapy module, thereby expanding the device's applicability for comprehensive facial or oral care.

The incorporation of a load detection or orientation sensing module provides intelligent state recognition, allowing automatic adjustment of operating modes based on positional alignment or user interaction. This feature enhances operational safety by ensuring that light emission occurs only when the device is correctly positioned and properly connected. It also simplifies user operation by eliminating the need for manual switching between modes.

The improved coupling structure, featuring magnetically assisted alignment or mechanical interlocking elements, offers a stable and user-friendly assembly experience. The design minimizes movement and separation during use, thereby maintaining consistent treatment performance and user comfort. The integrated charging assembly further improves convenience, allowing for efficient energy replenishment, hygienic storage, and optional sterilization when not in operation.

The oral care device addresses the deficiencies of existing lip beautifiers and oral treatment devices by providing a stable, modular, and intelligent system capable of performing diverse cosmetic and therapeutic functions. The invention not only enhances the performance and usability of light-based oral care products but also contributes to improved user experience, safety, and treatment efficacy.

Embodiments of the present invention will now be described with reference to the **FIGS.**

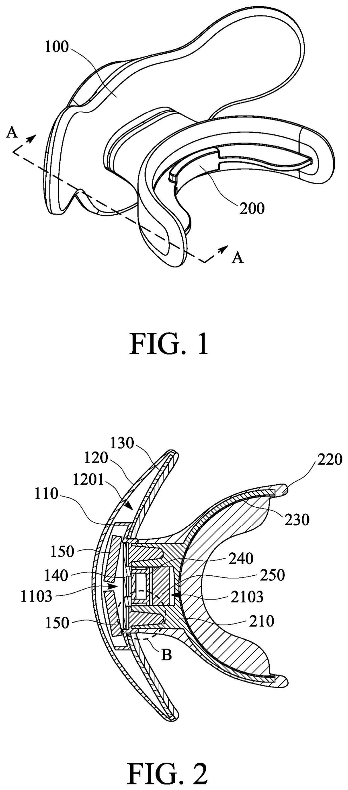

Referring to **FIG. 1** to **FIG. 11****,** the present application provides a mouth aesthetic device comprising a lip care assembly 100 and an occlusal structure configured for tooth engagement and support. The lip care assembly 100 includes a first mount, a first phototherapy part, a first support for supporting the occlusal structure, and a first electrical contact 141 electrically connected to the first phototherapy part. The first mount has a light output side, and the first phototherapy part is installed on the first mount with its luminous side oriented toward the light output side. Both the first support and the first electrical contact 141 are arranged on the light output side of the first mount. The occlusal structure includes a second support configured for detachable connection with the first support.

Specifically, the first mount serves to support and position the first phototherapy part. During use, the light output side of the first mount faces the user's lips, thereby positioning the luminous side of the first phototherapy part toward the lips. The luminous surface of the first phototherapy part emits light of specific wavelengths suitable for lip beautification. This light irradiates the user's lips to stimulate collagen formation, promote blood circulation, and enhance the firmness, smoothness, and overall appearance of the lips.

The occlusal structure is a component adapted to be placed inside the user's mouth and clamped between the upper and lower rows of teeth. When in use, the occlusal structure is fixed to the light output side of the first mount through the detachable engagement between the second support and the first support. By biting onto the occlusal structure, the user holds the lip care assembly 100 stably in position at the lips, thereby allowing the first phototherapy part to perform lip beautification treatment effectively and comfortably.

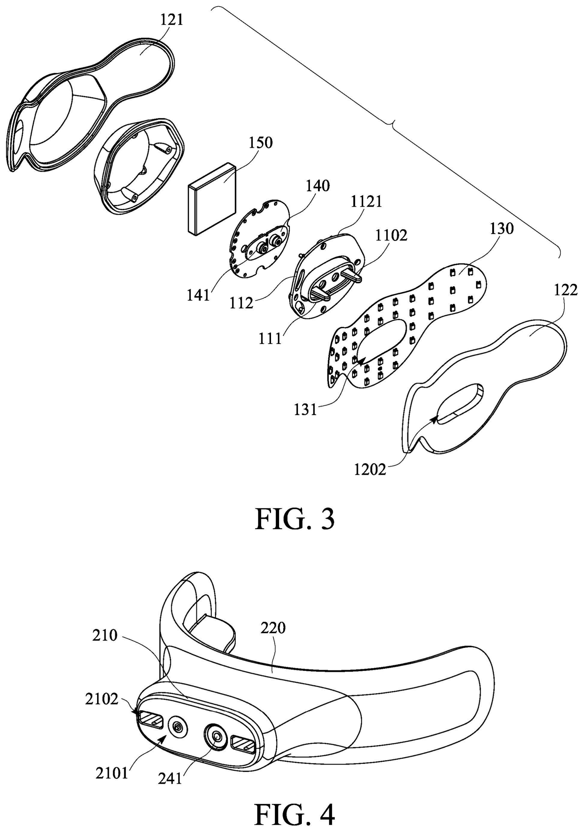

Referring to **FIG. 2** and **FIG. 3****,** in some embodiments, the first mount comprises a main body portion 120 and a first bracket 110 installed within the main body portion 120. The first phototherapy component is positioned inside the main body portion 120 and is mounted on the first bracket 110. One end of the first bracket 110 is exposed on the light output side and is configured to engage with an end of the occlusal structure. The first support and the first electrical contact 141 are disposed at the exposed light output end of the first bracket 110.

Specifically, the first bracket 110 supports the first phototherapy component within a first cavity 1201 formed in the main body portion 120. The shape of the first bracket 110 is configured according to the connection structure of the first phototherapy component. The main body portion 120 is generally made of a medical-grade silicone material, and its shape corresponds to the contour of the first bracket 110 and the first phototherapy component, thereby forming the first phototherapy component within the first cavity 1201 for protection. The light output side of the main body portion 120 is made of transparent or translucent material so that light emitted by the first light panel 130 can pass through and irradiate the user's lips.

In an embodiment, the main body portion 120 also exhibits anti-contamination properties, making it easy to clean and disinfect to ensure hygiene and safety during repeated use. Additionally, the main body portion 120 functions as a waterproof seal, thereby improving overall operational safety and durability of the lip care assembly.

In one example, the first bracket 110 comprises a first lip support member 111 and a second lip support member 112. The first lip support member 111 is connected at one end of the second lip support member 112. The end face area of the second lip support member 112 facing the first lip support member 111 is greater than the cross-sectional area of the first lip support member 111, thereby forming a positioning surface 1121 at the interface between the two members. The first lip support member 111 and the second lip support member 112 are integrally molded or independently fabricated and fixed together by bonding or connecting elements. The end of the first lip support member 111 opposite the second lip support member 112 defines a first support portion 1101, which is shaped to tooth care assembly correspond to the end of the occlusal structure for connection.

The first support is formed as a support column 1102 protruding from the first lip support member 111. The cross-sectional shape of the support column 1102 is one of a rectangular, circular, or another configuration. The number of first supports may vary; for example, two parallel support columns may be provided. Alternatively, the first support may be formed as a support groove recessed into the first lip support member 111.

The main body portion 120 comprises an outer silicone sleeve 121 and an inner silicone sleeve 122. The inner silicone sleeve 122 is inserted within the outer silicone sleeve 121, thereby defining the first cavity 1201 between them. The inner silicone sleeve 122 includes a first opening 1202 shaped to correspond to the first lip support member 111. During assembly, the first phototherapy component is first mounted onto the first bracket 110, the first opening 1202 of the inner silicone sleeve 122 is then fitted over the first bracket 110, and finally, the outer silicone sleeve 121 is wrapped around the second lip support member 112 of the first bracket 110. The inner silicone sleeve 122 and the outer silicone sleeve 121 are fixed together by bonding or similar means, thereby encapsulating the first phototherapy component within the first cavity 1201, with the first support portion 1101 of the first bracket 110 exposed on the light output side. This structure facilitates a connection between the first support and the corresponding second support on the occlusal structure. Alternatively, the main body portion 120 is integrally formed, with the first light panel 130 and related components inserted into the first cavity 1201 by temporarily expanding the first opening 1202 through the inherent elasticity of the silicone material.

In some embodiments, the first phototherapy component comprises a first light panel 130, a first phototherapy circuit 140 for controlling the first light panel 130, and a first battery 150 electrically connected to the first phototherapy circuit 140. The system further includes a second electrical contact 241 electrically connected to a second circuit board. Specifically, the first light panel 130 comprises a substrate on which a plurality of LED lamp beads are evenly arranged. The luminous surface of the first light panel 130 corresponds to the side where the LED lamp beads are disposed. Particularly, the LEDs are configured to emit light in the wavelength range of 600 nm to 700 nm (red light) or 750 nm to 1100 nm (infrared light) or in combination, suitable for stimulating collagen production and promoting blood circulation to enhance the cosmetic appearance of the user's lips. The shape of the first light panel 130 is either arcuate or otherwise contoured to match the user's lip shape, thereby improving coverage and fit for enhanced treatment comfort and efficiency.

A through hole 131 having a cross-sectional shape corresponding to that of the first lip support member 111 is provided on the first light panel 130. A set of through holes 131 is arranged along the first lip support member 111. The rear surface of the first light panel 130, opposite its luminous surface, abuts the positioning surface 1121 to define its installation position. The back surface of the first light panel 130 is further secured to the positioning surface 1121 by bonding or adhesive means to maintain stable positioning during operation. When the first light panel 130 is mounted on the first lip support member 111 and positioned against the positioning surface 1121, the first support portion 1101 is located on the front side of the luminous surface of the first light panel 130, ensuring that the light-emitting surface faces outward toward the light output side.

The first phototherapy circuit 140 is a printed circuit board integrating various electronic components such as a microcontroller, LED driver, and control circuitry for the first light panel 130. The first battery 150 is a rechargeable lithium-ion battery with high energy density and long service life, capable of providing stable and continuous power to the lip care assembly 100. The first phototherapy circuit 140, first battery 150, and first light panel 130 are electrically connected via conductive wiring. The first battery 150 supplies power to both the first phototherapy circuit 140 and the first light panel 130, while the first phototherapy circuit 140 controls the switching, irradiation time, and light intensity of the first light panel 130.

The first bracket 110 defines a first mounting cavity 1103, which is a hollow space of predetermined volume. The second lip support member 112 of the first bracket 110 includes an opening communicating with the first mounting cavity 1103 on the side of the first lip support member 111. The first phototherapy circuit 140 and the first battery 150 are sequentially installed within the first mounting cavity 1103 to ensure their stability and protection during use of the lip care assembly 100.

The first electrical contact 141 is made of a conductive metal material. It may be directly welded to the surface of a second phototherapy circuit 240 or connected to the first phototherapy circuit 140 via a conductive wire. The shape of the first electrical contact 141 may vary; for instance, it may be cylindrical. The number of first electrical contact 141 may also vary; for example, two electrodes/electrical contacts may be arranged in parallel, corresponding respectively to the positive and negative terminals of the control circuit on the first phototherapy circuit 140. When the first phototherapy circuit 140 is mounted within the first mounting cavity 1103, the first support portion 1101 of the first lip support member 111 is provided with mounting holes corresponding in number to the first electrical contact 141. The first electrical contact 141 is inserted through these mounting holes such that they are exposed at the first support portion 1101, with their rear sides facing the first phototherapy circuit 140.

Referring to **FIG. 1** to **FIG. 6****,** in certain embodiments, the occlusal structure is configured as a tooth care assembly 200, which includes a second mount, a second phototherapy component for performing phototherapy on the user's teeth, and a second electrical contact 241 electrically connected to the second phototherapy component. The second phototherapy component is mounted within the second mount, and both the second electrical contact 241 and the second support are disposed at one end of the second mount adjacent to the first mount. The second electrical contact 241 is configured to come into electrical contact with the first electrical contact 141 when the first support is coupled to the second support, thereby enabling electrical conduction between the lip assembly and the dental assembly.

In operation, the tooth care assembly 200 functions to enhance the cosmetic appearance of the user's teeth. During use, the user inserts the portion of the second mount into the mouth such that the luminous surface of the second phototherapy component faces the tooth surface. The luminous surface emits light of a specific wavelength directed toward the teeth, which can activate whitening agents present on the tooth surface and reduce discoloration or pigmentation, thereby improving overall tooth brightness and color.

Through the detachable connection of the first support and second support, the first mount and second mount can be securely joined, enabling the lip care assembly 100 and tooth care assembly 200 to be stably fixed together. When the user places the tooth care assembly 200 in the mouth, the lip care assembly 100 is simultaneously positioned against the lips. Furthermore, by virtue of the electrical contact between the first electrical contact 141 and the second electrical contact 241, the first phototherapy component and the second phototherapy component can be synchronously powered and controlled. This configuration allows for simultaneous operation of both the lip care assembly 100 and the tooth care assembly 200, thereby improving overall treatment efficiency and user experience.

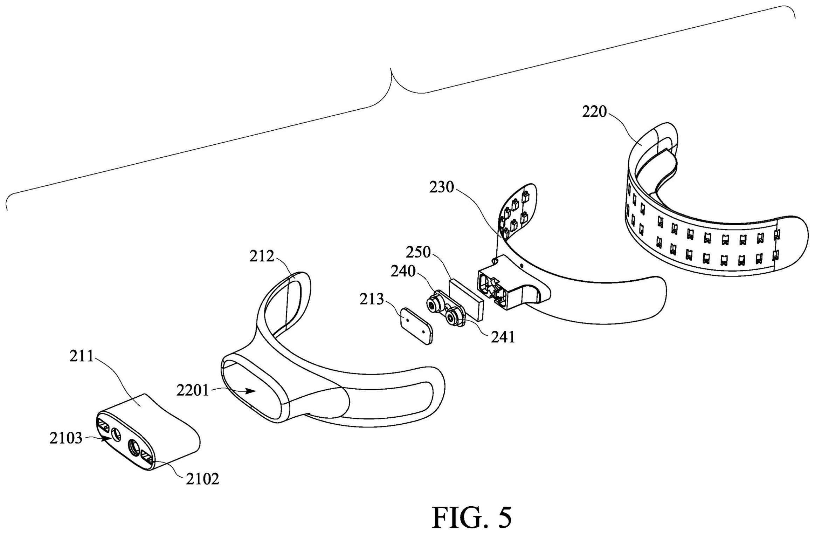

Referring to **FIG. 4** and **FIG. 5****,** in certain embodiments, the second mount comprises an occlusal sleeve 220 and a second bracket 210 disposed within the occlusal sleeve 220. The second phototherapy component is mounted inside the occlusal sleeve 220 and is connected to the second bracket 210. One end of the second bracket 210 extends through the occlusal sleeve 220 toward the first bracket 110, and both the second support and second electrical contact 241 are arranged at this exposed end of the second bracket.

In one example, the second bracket 210 includes a first tooth support 211 and a second tooth support 212, with the first tooth support 211 connected to one side of the second tooth support 212. The second tooth support 212 has an arcuate shape corresponding to the contour of the user's dental arch. The first tooth support 211 is positioned outward of the second tooth support 212, and the surface of the first tooth support 211 facing away from the second tooth support 212 defines a second support portion 2101. The first tooth support 211 and the second tooth support 212 can be integrally molded or separately manufactured and fixed together using bonding or mechanical fastening means.

When the first support is configured as a support column 1102 protruding from the first bracket 110, the second support is designed as a support groove 2102 on the second bracket 210 for receiving the support column 1102. The shape of the support groove 2102 corresponds to that of the support column 1102, ensuring a secure and stable insertion fit. The number of second supports corresponds to the number of first supports; for instance, two second supports are provided to align with two first supports in a one-to-one configuration. Conversely, when the first support is designed as a recessed groove on the first bracket 110, the second support is configured as a convex column on the second bracket 210.

The occlusal sleeve 220 may be formed of medical-grade silicone and can be integrally molded. A second cavity 2201 is defined within the occlusal sleeve 220 to accommodate the shape of the second bracket 210 and the second light panel 230, with a second opening facing toward the first bracket 110. During assembly, the elasticity of the occlusal sleeve 220 allows the second opening to expand, enabling insertion of the entire second bracket 210 and the second phototherapy assembly into the second cavity 2201. The second support portion 2101 of the first tooth support 211 extends through the second opening, exposing the second support and the second electrical contact 241 on the outer surface of the occlusal sleeve 220, thereby facilitating a detachable electrical and mechanical connection between the tooth care assembly 200 and the lip care assembly 100. The occlusal sleeve 220 may also be configured as a split or multi-part assembly.

During use, when the user inserts the tooth care assembly 200 into the mouth, the occlusal sleeve 220 contacts the inner surface of the oral cavity and the tooth surfaces, improving comfort and stability. The second phototherapy component, being encapsulated within the second cavity 2201, is sealed and waterproofed by the occlusal sleeve 220, enhancing safety during operation. At least the portion of the occlusal sleeve 220 corresponding to the second light panel 230 is made of a transparent or translucent material to allow the emitted light to pass through and reach the tooth surfaces.

The occlusal sleeve 220 further includes a biting portion 221 on the side facing the user's teeth. The biting portion 221 can be shaped as a crescent to conform to the dental structure. A limiting portion 222 is also provided on the biting portion 221, which is a raised structure positioned on the side opposite the second light panel 230 and protruding on both the upper and lower surfaces of the biting portion 221. The spacing between the limiting portion 222 and the luminous surface of the second light panel 230 corresponds to the thickness of the user's teeth, forming a limiting slot 2202 in which the teeth can be seated.

When the user bites onto the biting portion 221, the tooth care assembly 200 is securely positioned within the mouth. The limiting portion 222, being positioned along the inner side of the teeth, prevents outward displacement of the tooth care assembly 200. This configuration allows the user to maintain the device in position without applying continuous biting force, improving comfort and reducing fatigue during prolonged use.

In some embodiments, the second phototherapy component comprises a second light panel 230 and a second phototherapy circuit 240 for controlling the light emission, with the second electrical contact 241 electrically connected to the second phototherapy circuit 240. The second light panel 230 consists of a substrate populated with multiple LED lamp beads evenly arranged on its surface. The luminous surface corresponds to the side bearing the LEDs, which emit light in the wavelength range of 400 nm to 500 nm for tooth whitening. The arc-shaped configuration of the second light panel 230 allows it to conform closely to the curvature of the user's dental arch, providing full coverage and optimal contact for improved comfort and uniform illumination.

The back surface of the second light panel 230 (opposite its luminous surface) can be secured to the inner side of the second tooth support 212 through bonding or equivalent means, enabling the second tooth support 212 to structurally support the second light panel 230. When positioned inside the mouth, the second light panel 230 is located between the lips and teeth.

The second phototherapy circuit 240 is a printed circuit board incorporating electronic control components such as a microcontroller, LED driver, and control circuitry for regulating the second light panel 230. The second light panel 230 and second phototherapy circuit 240 are electrically connected via wiring to enable control of illumination parameters such as activation, deactivation, irradiation duration, and intensity.

A second mounting cavity 2103 is provided in the second bracket 210, preferably in the first tooth support 211, to accommodate the second phototherapy circuit 240. The opening of the second mounting cavity 2103 is formed at the second support portion 2101, with the two second supports disposed on either side of the cavity. The second phototherapy circuit 240 is secured within the cavity to ensure stability during use. Additionally, the second bracket 210 includes a cover plate 213 having a shape corresponding to the cavity opening, which is used to close the cavity and protect the second phototherapy circuit 240 from exposure or damage.

The second electrical contact 241 may be formed of a conductive metal material and can either be soldered directly to the first phototherapy circuit 140 or connected to the second phototherapy circuit 240 via wires. The shape of the second electrical contact 241 may vary, for example, cylindrical, and its number corresponds to that of the first electrical contact 141. In one embodiment, two parallel second electrical contacts 241 are provided, each corresponding respectively to the positive and negative terminals of the control circuit on the second phototherapy circuit 240. When installed, the cover plate 213 is formed with second mounting holes corresponding in number to the second electrical contact 241. Each second electrical contact 241 passes through a mounting hole such that its end surface is exposed at or slightly protrudes from the second support portion 2101, ensuring reliable electrical contact with the corresponding first electrical contact 141 during coupling of the lip and dental assemblies.

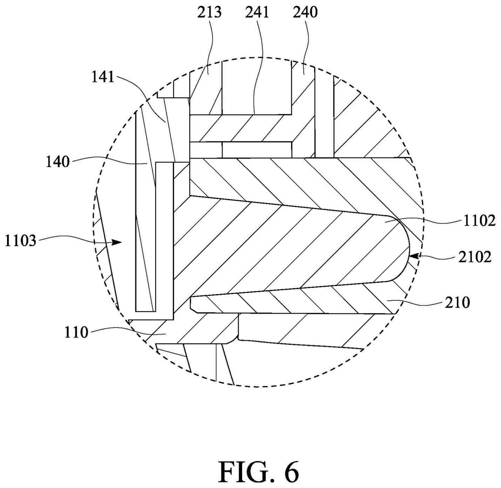

Referring to **FIG. 6****,** when the lip care assembly 100 and the tooth care assembly 200 are connected through engagement between the first support and the second support, the first support portion 1101 and the second support portion 2101 are aligned and positioned in proximity. At the same time, the first electrical contact 141 and the second electrical contact 241 are positioned opposite each other and come into conductive contact, thereby establishing an electrical connection between the first phototherapy circuit 140 and the second phototherapy circuit 240.

Through this electrical interface, the first electrical contact 141 and the second electrical contact 241 enable bidirectional conduction and coordination between the two circuit systems. On one hand, the first phototherapy circuit 140 and the second phototherapy circuit 240 can operate synchronously, allowing coordinated control of both the lip phototherapy and dental phototherapy functions. On the other hand, the first battery 150 housed within the lip care assembly 100 can also serve as a shared power source, supplying power to the second phototherapy circuit 240 and the second light panel 230, thereby enabling the first phototherapy module and second phototherapy module to function cooperatively and simultaneously.

In certain embodiments, the first electrical contact 141 is positioned between the two first supports, while the second electrical contact 241 is correspondingly positioned between the two second supports. This structural alignment ensures stable electrical contact between the first electrical contact 141 and the second electrical contact 241, minimizing the risk of disconnection or misalignment during use and enhancing the reliability of the connection.

Additionally, the second phototherapy component further comprises a second battery 250, which is housed within the second mounting cavity 2103 along with the second phototherapy circuit 240. The second battery 250 provides dedicated power to the second phototherapy circuit 240 and the second light panel 230, allowing the tooth care assembly 200 to operate independently when detached from the lip care assembly 100, thereby offering flexible usage options for the user.

In an embodiment of the present invention, the first phototherapy circuit and/or the second phototherapy circuit includes a micro-current element, an electrotherapy element, a heating element, a cooling element, or a vibration element, either present individually or in combination.

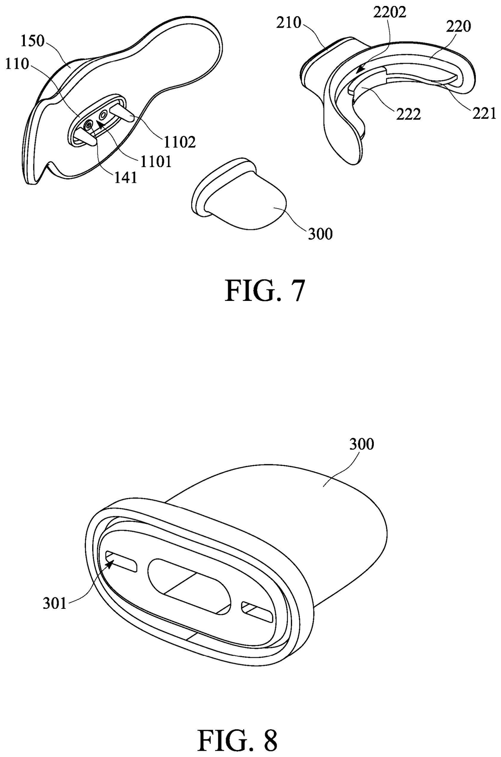

Referring to **FIG. 7** to **FIG. 9****,** in certain embodiments, the oral beauty device further includes a mouthpiece 300, which is configured to detachably connect with the lip care assembly 100 through engagement between the first support and the second support. The second support is provided on the mouthpiece 300, enabling the mouthpiece to be easily attached to or detached from the lip beauty assembly as needed.

In some examples, the mouthpiece 300 is generally flat or contoured to accommodate comfortable biting by the user. The thickness of the mouthpiece 300 can be customized or varied to match different users' oral structures, including tooth alignment, mouth size, and individual bite force, thereby ensuring proper fit and comfort during use. The mouthpiece 300 is formed from a high-hardness silicone material, leveraging silicone's flexibility, biocompatibility, and resilience to conform to the user's oral cavity while maintaining structural stability. This configuration provides a comfortable yet firm bite feel, improving user experience during extended use.

Additionally, the mouthpiece 300 can include hollowed regions or internal cavities to reduce overall weight, minimizing fatigue during prolonged operation while maintaining sufficient mechanical strength to support the lip care assembly 100.

In one embodiment, when the first support is a support column 1102 protruding from the first bracket 110, the second support on the mouthpiece 300 is formed as a second support groove 301 recessed therein. The support column 1102 can be inserted into the second support groove 301 to achieve stable mechanical coupling, thereby fixing the mouthpiece 300 to the first support portion 1101 of the first bracket 110. Once connected, the user can insert the mouthpiece 300 into the mouth, enabling the lip care assembly 100 to be independently supported and positioned against the user's lips for targeted phototherapy treatment.

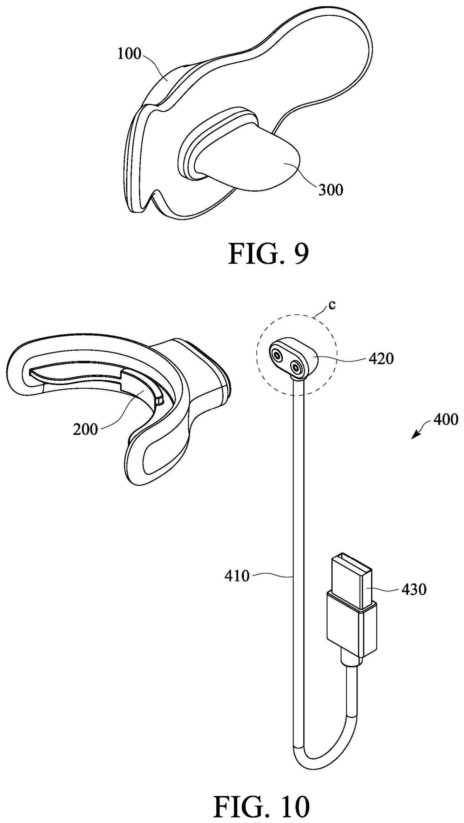

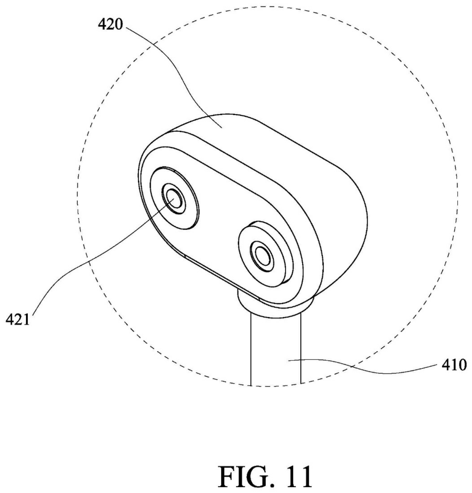

Referring to **FIG. 10** and **FIG. 11****,** in certain embodiments, the mouth beauty device further includes a charging assembly 400 configured to supply electrical power to the device. The charging assembly 400 comprises a charging lead 410, a charging connector 430, and a magnetic connector 420, wherein the charging connector 430 and the magnetic connector 420 are respectively connected to opposite ends of the charging lead 410. The magnetic connector 420 is provided with a third electrode 421, which is configured to establish a magnetic and electrical connection with either the first electrical contact 141 or the second electrical contact 241 for charging purposes.

In some embodiments, the charging connector 430 is a USB connector, enabling the mouth beauty device to be powered or recharged via a standard USB charger or a USB port of an electronic device, such as a computer or power bank. Alternatively, the charging connector 430 can be a Type-C connector or a Lightning connector, thereby offering compatibility with various charging interfaces and enhancing convenience for users with different electronic devices.

The third electrode 421 is preferably made from a magnetically conductive material, such as a nickel-copper alloy, stainless steel, or ferrite, which enables stable magnetic attraction to the first electrical contact 141 or the second electrical contact 241. Both the first and second electrical contacts are likewise formed from metallic materials capable of magnetic coupling. When magnetically connected, the third electrode 421 establishes electrical conduction between the charging connector 430 and the first phototherapy circuit 140 or second phototherapy circuit 240, allowing electrical energy to be delivered from an external charger to the first battery 150 for recharging.

In an embodiment of the present invention, the first electrical contact and the second electrical contacts comprise pogo pins.

In configurations where the second phototherapy component does not include an independent second battery 250, the charging assembly 400 can remain connected to the tooth care assembly 200. In this state, continuous power supply can be provided to the second phototherapy circuit 240 and the second light panel 230, thereby enabling the tooth care assembly 200 to operate independently while maintaining stable power delivery through the charging component.

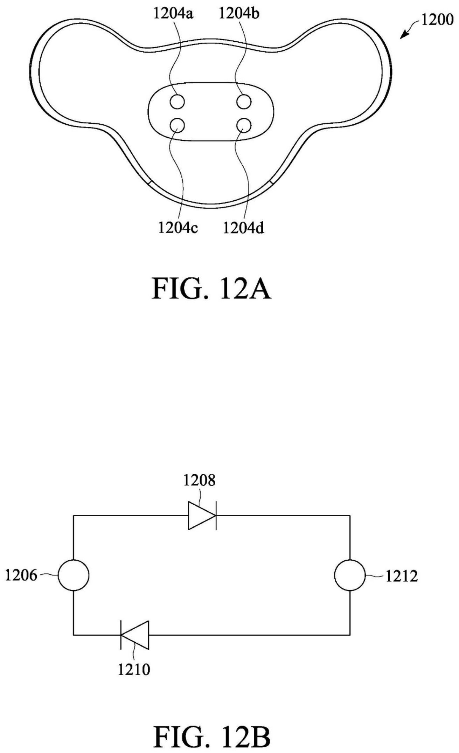

**FIGS. 12A and 12B** illustrate an exemplary load and orientation detection module incorporated into the oral care device to detect coupling orientation and to control operational modes of the tooth care assembly relative to the lip care assembly.

In one embodiment, as shown in **FIG. 12A****,** a detection interface 1200 is disposed on a surface of the lip care assembly that faces the tooth care assembly during use. The detection interface 1200 includes a plurality of electrodes 1204a, 1204b, 1204c, and 1204d that are arranged in predetermined positions to enable selective electrical engagement with corresponding connection terminals provided on the tooth care assembly. The electrodes 1204a-1204d function as polarity and signal terminals for two separate light-emitting circuits within the tooth care assembly, denoted as LED1 and LED2. In the illustrated embodiment, electrodes 1204a and 1204b correspond respectively to LED1- and LED1+, while electrodes 1204c and 1204d correspond to LED2- and LED2+. The electrodes may be formed of a conductive metallic material such as gold-plated copper or stainless steel, and may be embedded within an insulating elastomeric substrate of the lip care assembly to ensure stable electrical contact and user comfort.

In In one embodiment, the LED1 circuit serves as the first phototherapy circuit, located within the lip care assembly for treating the lips, while the LED2 circuit serves as the second phototherapy circuit, located within the tooth care assembly for treating the teeth.

**FIG. 12B** illustrates a corresponding detection and control circuit disposed in the tooth care assembly. The circuit includes a first connection terminal 1206 and a second connection terminal 1212, which are configured to selectively contact the electrode pairs of the lip care assembly depending on the assembly's orientation during coupling. A first light-emitting diode (LED1) 1208 and a second light-emitting diode (LED2) 1210 are connected in parallel branches between the terminals (1206 and 1212). Each of the diodes 1208 and 1210 represents an operational mode of the tooth care assembly, corresponding respectively to a first phototherapy mode and a second phototherapy mode. The circuit is designed such that when the terminals (1206 and 1212) engage the LED1- and LED1+ electrodes (1204a, 1204b) of the lip care assembly, current flows through LED1 1208 to activate a first phototherapy configuration, while LED2 1210 remains inactive. Conversely, when the tooth care assembly is coupled in a flipped orientation such that the terminals (1206 and 1212) engage the LED2- and LED2+ electrodes (1204c, 1204d), current flows through LED2 1210, activating the second phototherapy configuration while LED1 1208 remains off.

The lip care assembly may further include a load detection module electrically coupled to the electrode array 1204a-1204d. The module is configured to sense electrical load or potential across the electrodes to determine whether a connection is established with LED1 or LED2 of the tooth care assembly. Based on the detected load state, the module transmits a control signal to the at least one controller of the oral care device to identify the current operational orientation of the coupled assemblies. The controller may then adjust or synchronize phototherapy parameters such as wavelength output, light intensity, duty cycle, or treatment timing between the lip and tooth care assemblies.

In another embodiment, the LED1 and LED2 are positioned within the second phototherapy circuit, either in the upper and lower tooth trays or arranged side by side. The load detection module, upon detecting the orientation of the tooth tray, adjusts the operation mode of the LED1 and LED2 circuits, including activating or deactivating them, or modifies their emitted wavelength accordingly.

This arrangement enables the oral care device to automatically detect whether the tooth care assembly is in a first or second orientation, and to selectively control activation of the corresponding phototherapy circuit without requiring user intervention. The orientation detection is achieved through the inherent polarity and circuit configuration of the electrodes/electrical contacts and light-emitting diodes, eliminating the need for additional mechanical sensors or orientation switches. Furthermore, the system allows seamless transition between multiple operational modes of the tooth care assembly while maintaining independent or synchronized operation of the lip care assembly.

In some embodiments, the load detection module may include an analog front-end circuit or microcontroller configured to measure impedance, voltage drop, or current flow across the electrode terminals/electrical contacts. The controller may store threshold parameters corresponding to connection states, thereby enabling automatic recognition of assembly orientation and ensuring reliable mode switching. Optionally, the controller may disable power output in the event of incomplete or erroneous electrode/electrical contact, enhancing safety and device reliability.

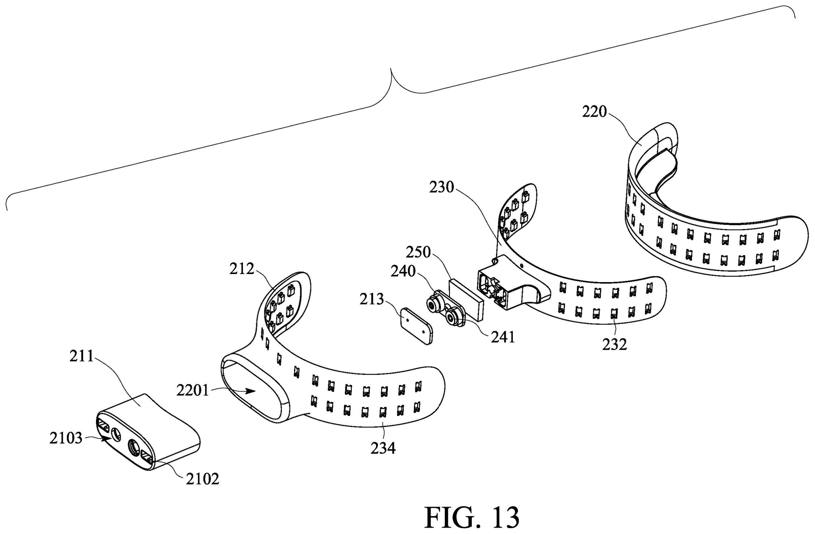

**FIG. 13** illustrates an exploded view of an exemplary configuration of the tooth care assembly showing the arrangement of multiple phototherapy components for emitting light toward both the teeth and the inner surface of the lips. The tooth care assembly comprises an outer surface 234 on the second tooth support 212 arranged to face the inner side of the lips when the device is in use. The second light panel 230 includes plurality of LEDs for emitting therapeutic radiation on inner surface as well as an external surface 232. In the illustrated configuration, the LEDs on the external surface 232 are positioned along the outer surface 234 of the second tooth support 212 and are configured to irradiate the inner surface of the lips, thereby promoting collagen activation, tissue rejuvenation, and inner-lip treatment. Simultaneously, the inner LEDs positioned on the inner surface are configured to emit light toward the user's teeth, providing phototherapy for whitening, stain removal, or general dental cosmetic enhancement. The distribution of LEDs on both sides of the second light panel 230 ensures uniform optical coverage of oral and lip tissues during operation.

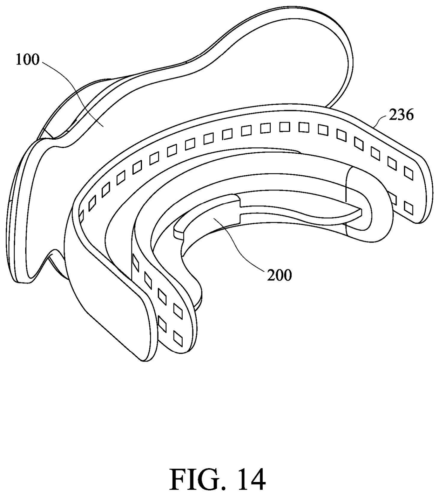

**FIG. 14** illustrates the oral care device having an intermediate phototherapy layer 236 to irradiate inner surface of lips. The intermediate phototherapy layer 236 is disposed or positioned between the lip care assembly and the tooth care assembly. The intermediate phototherapy layer comprises an additional array of light-emitting diodes or optical waveguides configured to emit light at a wavelength suitable for stimulating inner-lip tissues or for treating oral mucosa. The intermediate layer may be made of a flexible transparent substrate embedded with LEDs or light-transmitting optical fibers, and it may be detachable or replaceable to allow different wavelength configurations or treatment intensities as required.

The intermediate phototherapy layer 236 is provided as a standalone component positioned between the lip care assembly and the tooth care assembly when the device is assembled for use. In this configuration, the intermediate phototherapy layer 236 may include its own phototherapy circuit and electrical contacts configured to interface with those of the lip and tooth care assemblies. This arrangement enables the intermediate phototherapy layer 236 to emit light toward the inner surface of the lips while maintaining simultaneous operation of the lip and tooth care phototherapy systems. The independent or integrated configuration of the intermediate layer thus enhances treatment versatility, allowing the device to perform comprehensive outer-lip, inner-lip, and dental phototherapy simultaneously or selectively.

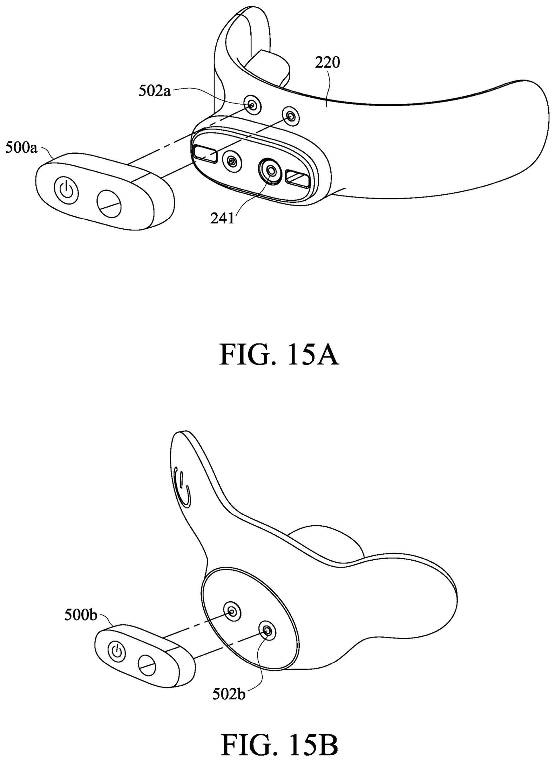

**FIGS. 15A** and **15B** illustrate exemplary configurations of a control system for an oral care device, showing detachable control units configured to manage the operation of the lip care assembly and the tooth care assembly, either independently or in synchronized control.

Referring to **FIG. 15A****,** the tooth care assembly includes a second mount provided with one or more second electrical contacts 241 positioned at its exposed end. A control unit 500a is detachably connectable to the tooth care assembly via a corresponding connection interface 502a. The control unit 500a may include an internal controller, a power supply, and one or more user-operable input elements such as a power button or mode selector. When attached, the control unit 500a supplies electrical energy to the second phototherapy circuit and provides control signals to regulate light emission parameters such as wavelength, duration, and intensity. The detachable configuration allows the control unit to be easily removed for charging, replacement, or sterilization while maintaining the compact form factor of the oral care device during use.

Referring to **FIG. 15B****,** a similar arrangement is shown for the lip care assembly. The lip care assembly includes a connection port 502b configured to receive a corresponding wired or wireless detachable control unit 500b. The control unit 500b likewise contains a controller and power supply configured to operate the first phototherapy circuit within the lip care assembly. In certain embodiments, the control unit 500b may be integrated directly within the lip care assembly housing, eliminating the need for external attachment while maintaining water resistance and improved aesthetics. Further, the connection port 502b may serve as a charging terminal for recharging the battery or power source of the lip care assembly or the tooth care assembly..

In use, the oral care device may operate under several control modes. When the lip care assembly and the tooth care assembly are uncoupled, each control unit (500a, 500b) functions independently, allowing the user to operate either the lip treatment or the dental treatment separately. Each controller is configured to execute preprogrammed phototherapy modes corresponding to the respective treatment requirements, and each power supply independently energizes the associated phototherapy circuits.

When the lip care assembly and the tooth care assembly are mechanically and electrically coupled, the control units enter a synchronized or cooperative control mode. In this mode, communication between the electrical contacts (such as 241) establishes data and power linkage between the two assemblies, allowing coordinated operation of both phototherapy circuits. In one embodiment, the controller within the lip care assembly automatically assumes a master control role, while the controller within the tooth care assembly operates as a slave controller. The master controller sends synchronization and timing signals to the slave controller to ensure harmonized light emission patterns and uniform treatment timing across both assemblies.

The power management circuitry may further allow shared-power operation, wherein a single power supply, typically the one housed in the lip care assembly, can provide electrical energy to both assemblies when connected. This reduces overall device weight and simplifies charging requirements. The system may also include bidirectional charging capability, allowing either control unit to transfer power to the other when required, thus improving operational flexibility and energy efficiency.

In another embodiment, the two control units 500a and 500b may be integrated into a single centralized control unit configured to communicate wirelessly or through a wired connection with both the lip care assembly and the tooth care assembly. This configuration enables unified control of phototherapy parameters and simplifies user interaction by consolidating power management and mode selection in one module.

Furthermore, both control units may incorporate wireless communication modules such as Bluetooth^{®} or near-field communication (NFC) to facilitate synchronization, firmware updates, or remote control via a mobile application. Indicator lights or haptic feedback may also be provided on the control units to signal operational status, battery level, or synchronization state.

Accordingly, the configurations shown in **FIGS. 15A** and **15B** provide a versatile and modular control architecture that supports independent, coordinated, and master-slave operation of the oral care device, while also enabling detachable, rechargeable, and replaceable power and control modules. This design ensures flexibility in manufacturing and user customization, enhances portability, and allows for comprehensive and simultaneous lip and dental phototherapy treatment.

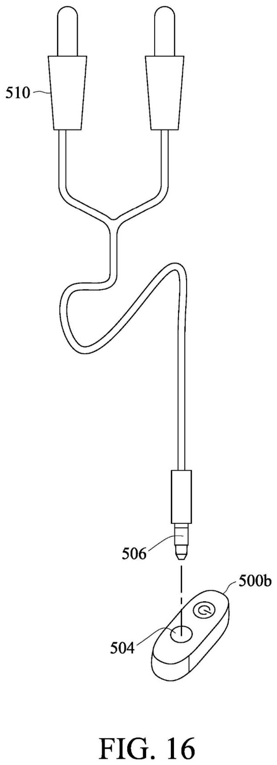

**FIG. 16** illustrates an embodiment of a nasal care phototherapy device that is connectable to a control unit for operation and power supply. The nasal care device 510 is configured to deliver targeted phototherapy to the nasal cavity for therapeutic, hygienic, or cosmetic benefits, such as promoting mucosal health, reducing inflammation, alleviating congestion, or enhancing blood circulation in the nasal region.

As shown, the nasal care device 510 includes a pair of light-emitting probes configured to be inserted partially into the user's nostrils. Each probe may house one or more light sources, such as light-emitting diodes (LEDs) or laser diodes, capable of emitting light in specific therapeutic wavelength ranges, for example, red, near-infrared, or blue light, depending on the intended treatment purpose. The light emitted from the probes provides localized photobiomodulation to the nasal mucosa, thereby stimulating tissue repair, reducing microbial load, and improving airflow and comfort.

The nasal care device 510 is electrically connected to the control unit 500b through a flexible connecting cable 504. The flexible connecting cable 504 may terminate in an audio-type auxiliary (AUX) connector 506, which mates with a corresponding input port on the control unit 500b. The AUX-type interface enables quick connection and disconnection while maintaining compactness and standardization across multiple treatment accessories. The control unit 500b, which may also be the same control unit used with the lip care assembly or tooth care assembly, provides electrical power and operational control to the nasal care device 510.

In some embodiments, the control unit 500b includes a built-in controller programmed with one or more treatment modes specific to nasal phototherapy. The user may initiate treatment via an input switch or touch-sensitive control provided on the control unit housing. The controller regulates the power output, pulse timing, wavelength selection, and overall treatment duration delivered to the nasal care device 510. An indicator light or display on the control unit may provide visual feedback regarding the operating state, battery level, or mode selection.

The system is designed for modular operation, allowing the nasal care device 510 to be used independently or in coordination with other treatment modules, such as the lip care assembly or the tooth care assembly. In synchronized operation mode, the control unit 500b may communicate with another control unit (for example, 500a) through a wired or wireless interface to achieve multi-region coordinated phototherapy. For instance, during a combined facial and nasal treatment session, the control unit can control timing and intensity parameters so that the nasal care device 510 operates in harmony with the light panels of the lip or dental modules.

In one embodiment, the nasal care device 510 may serve as an auxiliary attachment that receives both power and control signals from a master control unit associated with another treatment module. The controller may automatically recognize the attached accessory through electrical or magnetic coding and adjust the operational parameters accordingly. For example, when the nasal device is connected, the control unit may automatically switch to a preprogrammed "nasal therapy mode" that optimizes light pulse frequency and duration for intranasal application.

The modular architecture illustrated in **FIG. 16** enables flexible configuration of a comprehensive phototherapy system for oral and facial wellness. Through the use of a shared control platform and detachable auxiliary connectors, the user can interchange various phototherapy modules, such as lip, dental, or nasal devices, while utilizing a single power and control source. This design simplifies user experience, enhances portability, and supports future expansion of compatible therapeutic accessories without altering the base control electronics.

The oral care device disclosed herein is applicable in the field of personal care and beauty technology, particularly in oral aesthetic enhancement and light-based therapy. The device can be manufactured using commonly available medical-grade materials and standard electronic assembly processes, making it suitable for large-scale industrial production.

The invention provides a practical and user-friendly solution for improving lip and tooth appearance through integrated phototherapy modules. It can be widely used in cosmetic clinics, dental care centers, and home beauty treatments for teeth whitening, lip rejuvenation, and overall oral care enhancement. The detachable and modular structure allows convenient assembly, maintenance, and replacement of components, thereby increasing product lifespan and reducing manufacturing and maintenance costs.

Furthermore, the incorporation of standardized charging interfaces such as USB, Type-C, or Lightning connectors facilitates compatibility with existing consumer electronics infrastructure, promoting ease of use and broad market adoption.

Accordingly, the present invention demonstrates clear industrial applicability in the development and commercialization of beauty and oral-care devices, offering a safe, efficient, and scalable product suitable for mass production and consumer use.

Various modifications to these embodiments are apparent to those skilled in the art from the description and the accompanying drawings. The principles associated with the various embodiments described herein may be applied to other embodiments. Therefore, the description is not intended to be limited to the embodiments shown along with the accompanying drawings but is to provide the broadest scope consistent with the principles and the novel and inventive features disclosed or suggested herein. Accordingly, the invention is anticipated to hold on to all other such alternatives, modifications, and variations that fall within the scope of the present invention and appended claims.

## Claims

1. An oral care device comprising:
a lip care assembly including a first phototherapy circuit configured to emit light for cosmetic or therapeutic treatment of lips, and a first electrical contact;
a tooth care assembly including a second phototherapy circuit configured to emit light for cosmetic or therapeutic treatment of teeth, and a second electrical contact;
at least one control unit configured to control the operation of the oral care device; and
a detachable coupling structure configured to mechanically and electrically connect the lip care assembly and the tooth care assembly;
wherein electrical communication between the first electrical contact and the second electrical contact enables coordinated or independent operation of the first phototherapy circuit and the second phototherapy circuit.

2. The oral care device of claim 1, wherein the at least one control unit comprises:
a first controller and a first power supply associated with the lip care assembly; and
a second controller and a second power supply associated with the tooth care assembly.

3. The oral care device of claim 2, wherein:
the lip care assembly is configured to operate independently under the control of the first controller and the first power supply when uncoupled from the tooth care assembly;
the tooth care assembly is configured to operate independently under the control of the second controller and the second power supply when uncoupled from the lip care assembly; and
when the lip care assembly and the tooth care assembly are mechanically and electrically coupled, the first controller and the second controller are configured to operate in a coordinated phototherapy mode under synchronized control.

4. The oral care device of claim 2, wherein, when the tooth care assembly is coupled to the lip care assembly, the first controller automatically operates in a master-control mode to coordinate and control operation of the second controller and the second phototherapy circuit.

5. The oral care device of claim 1, further comprising a nasal therapy assembly configured to be detachably connected to the at least one of the lip care assembly or the tooth care assembly for cosmetic or therapeutic treatment of nasal tissue.

6. The oral care device of claim 1, wherein at least one of the control units is disposed within the lip care assembly or is detachably attachable to the lip care assembly.

7. The oral care device of claim 1, wherein the detachable coupling structure comprises:
a first support portion extending from the lip care assembly; and
a second support portion extending from the tooth care assembly and detachably connectable with the first support portion to mechanically couple the lip care assembly and the tooth care assembly;
the first electrical contact disposed on the lip care assembly and electrically connected to the first phototherapy circuit, and a second electrical contact disposed on the tooth care assembly and electrically connected to the second phototherapy circuit;
wherein, when the first support portion and the second support portion are connected, the first electrical contact and the second electrical contact engage to establish electrical communication between the lip care assembly and the tooth care assembly for synchronized or shared-power operation.

8. The oral care device of claim 7, wherein the first support portion comprises a support column, and the second support portion comprises a support groove configured to receive the support column to enable insertion and mechanical coupling between the lip care assembly and the tooth care assembly.

9. The oral care device of claim 7, wherein the at least one of the first support portion and the second support portion, or the first electrical contact and the second electrical contact includes a magnetic coupling structure configured to provide mechanical alignment and electrical connection between the lip care assembly and the tooth care assembly.

10. The oral care device of claim 7,
wherein the tooth care assembly comprises a second mount including an occlusal sleeve and a second bracket disposed within the occlusal sleeve;
the second bracket comprising a first tooth support and a second tooth support, and the second phototherapy circuit is supported by the second bracket.

11. The oral care device of claim 10, wherein an end of the second bracket is exposed from the occlusal sleeve adjacent the lip care assembly, and the second support portion and the second electrical contacts are positioned at the exposed end of the second bracket.

12. The oral care device of claim 10,
wherein the second phototherapy circuit is disposed between the occlusal sleeve and the second tooth support, and is configured to emit light toward the second tooth support to perform inner-lip treatment and to emit light toward the occlusal sleeve to perform tooth treatment.

13. An oral care device comprising:
a lip care assembly including a first light-emitting layer configured to emit light within a first wavelength range suitable for enhancing lip appearance;
a tooth care assembly including a second light-emitting layer configured to emit light within a second wavelength range suitable for treating teeth or oral tissue; and
an intermediate layer disposed between the lip care assembly and the tooth care assembly, the intermediate layer being positioned to contact or face an inner surface of lips and comprising a third light source configured to emit light having a wavelength effective for treating inner-lip tissue or sores;
wherein, during use, lips are positioned between the first light-emitting layer and the intermediate layer, enabling simultaneous irradiation of inner and outer lip surfaces.

14. The oral care device of claim 13, wherein the intermediate layer is disposed on the backside of the tooth care assembly, the backside being oriented toward an inner surface of the lips when the tooth care assembly is positioned within a mouth.

15. An oral care device comprising:
a lip care assembly including a first electrical contacts and a first phototherapy circuit;
a tooth care assembly including a second electrical contacts and a second phototherapy circuit; and
a load detection module electrically connected to at least one of the phototherapy circuits, the load detection module being configured to detect a positional state of the tooth care assembly relative to the lip care assembly based on electrical loading between corresponding first and second electrical contacts, and to selectively operate one or more of the phototherapy circuits according to the detected positional state.

16. The oral care device of claim 15, wherein the load detection module is configured to activate the first phototherapy circuit when the tooth care assembly is in a first orientation and to activate the second phototherapy circuit when the tooth care assembly is in a second orientation.

17. The oral care device of claim 15,
wherein the load detection module is configured to detect an orientation of the tooth care assembly and to control an operational mode of the first phototherapy circuit and the second phototherapy circuit based on a detected positional state of the tooth care assembly.
